# EUROPÄISCHE PATENTSCHRIFT

(11) **EP 1 337 254 B1**
(45) Veröffentlichungstag und Bekanntmachung des Hinweises auf die Patenterteilung: **19.01.2005**
(21) Anmeldenummer: 01998335.2
(22) Anmeldetag: 28.11.2001
(51) Int. Cl.: A61K 31/485, A61K 31/222, A61K 31/55, A61K 31/216, A61P 13/02

(54) **VERWENDUNG VON SCHWACHEN OPIOIDEN UND GEMISCHTEN OPIOIDAGONISTEN / -ANTAGONISTEN ZUR THERAPIE DER HARNINKONTINENZ**
USE OF WEAK OPIOIDS AND MIXED OPIOID AGONISTS/ANTAGONISTS FOR TREATING URINARY INCONTINENCE
UTILISATION D'OPIOIDES FAIBLES ET D'AGONISTES/D'ANTAGONISTES OPIOIDES MELANGES POUR TRAITER L'INCONTINENCE URINAIRE

(30) Priorität: 30.11.2000 DE 10059415
(43) Veröffentlichungstag der Anmeldung: 27.08.2003
(73) Patentinhaber: Grünenthal GmbH, 52078 Aachen (DE)
(72) Erfinder: CHRISTOPH, Thomas, 52080 Aachen (DE)
(86) Internationale Anmeldenummer: PCT/EP2001/013911
(87) Internationale Veröffentlichungsnummer: WO 2002/043713

(56) Entgegenhaltungen:
- EP-A- 0 913 152
- WO-A-93/15062
- DE-A- 19 947 747
- US-A- 5 354 863
- K. PARFITT: "Martindale - The complete drug reference - Thirty-second Edition" 1999 , PHARMACEUTICAL PRESS , LONDON UK XP002194480 Seite 26, Spalte 1 -Seite 27, Spalte 2 Seite 27, Spalte 3 -Seite 29, Spalte 1 Seite 34, Spalte 1 -Spalte 3 Seite 52, Spalte 2 -Seite 53, Spalte 2 Seite 89, Spalte 2
- MURRAY K.: "Acute urinary retention associated with sublingual buprenorphine" BRITISH MEDICAL JOURNAL, Bd. 286, Nr. 6367, 1983, Seiten 763-764, XP001064372
- MALINOVSKY J-M ET AL: "THE URODYNAMIC EFFECTS OF INTRAVENOUS OPIOIDS AND KETOPROFEN IN HUMANS" ANESTHESIA AND ANALGESIA, WILLIAMS AND WILKINS, BALTIMORE, MD, US, Bd. 87, Nr. 2, August 1998 (1998-08), Seiten 456-461, XP001064299 ISSN: 0003-2999 in der Anmeldung erwähnt
- FOWLER J E JR ET AL: "EFFECT OF LIQUID DIPHENOXYLATE HYDROCHLORIDE AND ATROPINE SULFATE LOMOTIL INSTILLATIONS OF DYNAMICS AND FUNCTION OF CONTINENT CECAL URINARY RESERVOIRS" JOURNAL OF UROLOGY, Bd. 138, Nr. 4, 1987, Seiten 735-738, XP001064958 ISSN: 0022-5347 in der Anmeldung erwähnt
- DATABASE PUBMED [Online] National Library of Medicine; PMID:9988096, 22. Januar 1999 (1999-01-22) ACETO M.D. ET AL.: "Stereoselective mu-and delta-opioid receptor-related antinociception and binding with (+)-thebaine" XP002200962 & ACETO MD ET AL.: "Stereoselective mu-and delta-opioid receptor-related antinociception and binding with (+)-thebaine" EUR. J. PHARMACOL., Bd. 365, Nr. 2-3, 22. Januar 1999 (1999-01-22), Seiten 143-147,
- DATABASE BIOSIS [Online] BIOSCIENCES INFORMATION SERVICE, PHILADELPHIA, PA, US; 1984 SPEIGEL K ET AL: "MEPTAZINOL A NOVEL MU-1 SELECTIVE OPIOID ANALGESIC" Database accession no. PREV198478013848 XP002200963 & JOURNAL OF PHARMACOLOGY AND EXPERIMENTAL THERAPEUTICS, Bd. 228, Nr. 2, 1984, Seiten 414-419, ISSN: 0022-3565
- ELWOOD W N: "Sticky business: patterns of procurement and misuse of prescription cough syrup in Houston." JOURNAL OF PSYCHOACTIVE DRUGS, (2001 APR-JUN) 33 (2) 121-33. REF: 57, XP001065228

## Beschreibung

Die Erfindung betrifft die Verwendung von schwachen Opioiden und gemischten Opioidagonisten / -antagonisten zur Herstellung eines Arzneimittels zur Behandlung von vermehrtem Harndrang bzw. Harninkontinenz sowie entsprechende Arzneimittel zur Behandlung von vermehrtem Harndrang bzw. Harninkontinenz.

Harninkontinenz ist der unwillkürliche Harnabgang. Dieser tritt unkontrolliert auf, wenn der Druck innerhalb der Harnblase den Druck übersteigt, der zum Schließen des Harnleiters notwendig ist. Ursachen können zum einen ein erhöhter interner Blasendruck (z. B. durch Detrusorinstabilität) mit der Folge der Dranginkontinenz und zum anderen ein erniedrigter Sphinkterdruck (z. B. nach Geburt oder chirurgischen Eingriffen) mit der Folge der Streßinkontinenz sein. Der Detrusor ist die grob gebündelte mehrschichtige Blasenwandmuskulatur, deren Kontraktion zur Harnentleerung führt, der Sphinkter der Schließmuskel der Harnröhre. Es treten Mischformen dieser Inkontinenzarten sowie die sogenannte Überflußinkontinenz (z. B. bei benigner Prostatahyperplasie) oder Reflexinkontinenz (z. B. nach Rückenmarksschädigungen) auf. Näheres dazu findet sich bei Chutka, D. S. und Takahashi, P. Y., 1998, Drugs 560: 587-595.

Harndrang ist der auf Harnentleerung (Miktion) abzielende Zustand vermehrter Blasenmuskelspannung bei Annäherung an die Blasenkapazität (bzw. bei deren Überschreitung). Dabei wirkt diese Anspannung als Miktionsreiz. Unter einem vermehrten Harndrang versteht man dabei insbesondere das Auftreten vorzeitigen oder gehäuften manchmal sogar schmerzhaften Harndrangs bis hin zum sog. Harnzwang. Das führt in der Folge zu einer deutlich häufigeren Miktion. Ursachen können u.a. Harnblasenentzündungen und neurogene Blasenstörungen sowie auch Blasentuberkulose sein. Es sind aber noch nicht alle Ursachen geklärt.

Vermehrter Harndrang wie auch Harninkontinenz werden als extrem unangenehm empfunden und es besteht ein deutlicher Bedarf bei von diesen Indikationen betroffenen Personen, eine möglichst langfristige Verbesserung zu erreichen.

Üblicherweise werden vermehrter Harndrang und insbesondere Harninkontinenz medikamentös mit Substanzen behandelt, die an den Reflexen des unteren Harntraktes beteiligt sind (Wein, A. J., 1998, Urology 51 (Suppl. 21): 43 - 47). Meistens sind dies Medikamente, die eine hemmende Wirkung auf den Detrusormuskel, der für den inneren Blasendruck verantwortlich ist, haben. Diese Medikamente sind z. B. Parasympatholytika wie Oxybutynin, Propiverin oder Tolterodin, trizyklische Antidepressiva wie Imipramin oder Muskelrelaxantien wie Flavoxat. Andere Medikamente, die insbesondere den Widerstand der Harnröhre oder des Blasenhalses erhöhen, zeigen Affinitäten zu a-Adrenorezeptoren wie Ephedrin, zu β-Adrenorezeptoren wie Clenbutarol oder sind Hormone wie Östradiol.

Auch bestimmte Diarylmethylpiperazine und -piperidine sind für diese Indikation in der WO 93/15062 beschrieben. Ebenso wurde für Tramadol ein positiver Effekt auf die Blasenfunktion in einem Rattenmodell rhythmischer Blasenkontraktionen nachgewiesen (Nippon-Shinyaku, WO 98/46216). Weiterhin gibt es in der Literatur Untersuchungen zur Charakterisierung der opioiden Nebenwirkung Harnretention, woraus sich einige Hinweise auf die Beeinflussung der Blasenfunktionen durch schwache Opioide wie Diphenoxylat (Fowler et al., 1987 J. Urol 138:735-738) und Meperidin (Doyle and Briscoe, 1976 Br J Urol 48:329-335), durch gemischte Opioidagonisten / -antagonisten wie Buprenorphin (Malinovsky et al., 1998 Anesth Analg 87:456-461; Drenger and Magora, 1989 Anesth Analg 69:348-353; Murray et al., 1983 Br. Med.J. 286: 763-764), Pentazocin (Shimizu et al. (2000) Br. J. Pharmacol. 131 (3): 610 - 616) und Nalbuphin (Malinovsky et al., 1998, a.a.O), sowie durch starke Opioide wie Morphin (Malinovsky et al., 1998 a.a.O; Kontani und Kawabata, (1988); Jpn J Pharmacol. Sep;48(1 ):31 ) und Fentanyl (Malinovsky et al., 1998 a.a.O) ergeben. Allerdings erfolgten diese Untersuchungen zumeist in analgetisch wirksamen Konzentrationen.

Bei den hier in Frage kommenden Indikationen ist aber zu beachten, daß es sich im allgemeinen um sehr langfristige medikamentöse Anwendungen handelt und sich die Betroffenen im Gegensatz zu vielen Situationen, in denen Analgetika eingesetzt werden, einer sehr unangenehmen, aber nicht unaushaltbaren Situation gegenüber sehen. Daher ist hier - noch mehr als bei Analgetika - darauf zu achten, Nebenwirkungen zu vermeiden, will der Betroffene nicht ein Übel gegen das andere tauschen. Auch sind bei einer dauerhaften Harninkontinenzbehandlung auch analgetische Wirkungen weitgehend unerwünscht.

Aufgabe der vorliegenden Erfindung war es daher, Stoffe aufzufinden, die zur Behandlung von vermehrtem Harndrang bzw. Harninkontinenz hilfreich sind und bei den wirksamen Dosen bevorzugt gleichzeitig geringere Nebenwirkungen und/oder analgetische Wirkungen zeigen.

Überraschenderweise wurde nun gefunden, daß bestimmte schwache Opioide und gemischte Opioidagonisten/-antagonisten bereits bei geringen Konzentrationen eine hervorragende Wirkung auf die Blasenfunktion besitzen und demzufolge gut zur Behandlung entsprechender Erkrankungen geeignet sind.

Dementsprechend ist Erfindungsgegenstand die Verwendung einer der folgenden Verbindungen
■ Codein,
■ Meptazinol oder
■ Tilidin
als freie Base und/oder in Form physiologisch verträglicher Salze zur Herstellung eines Arzneimittels zur Behandlung von vermehrtem Harndrang bzw. Harninkontinenz.

Überraschenderweise hatte sich herausgestellt, daß diese bekannten Verbindungen physiologische Parameter, die bei vermehrtem Harndrang bzw. Harninkontinenz von Bedeutung sind, deutlich positiv beeinflußen, was insbesondere an einer Verringerung des Intervalls in einem Modell mit rhythmischer Blasenkontraktion zu erkennen ist. Diese Veränderung kann eine deutliche Erleichterung im symptomatischen Bild betroffener Patienten bedeuten.

Geeignete Salze im Sinne dieser Erfindung und in jeder der beanspruchten Verwendungen sind Salze des jeweiligen Wirkstoffes mit anorganischen bzw. organischen Säuren und/oder einem Zuckeraustauschstoff wie Saccharin, Cyclamat oder Acesulfam. Besonders bevorzugt ist jedoch das Hydrochlorid.

Codein ((5 a ,6 a)- 7 ,8- di dehydro- 4 ,5- epoxy- 3- methoxy- 17- methyl morphinan- 6- ol) ist ein Wirkstoff mit antitussiver, narkotischer und analgetischer Wirkung. Er ist im deutschen Reichspatent (DRP) 247 180 von C. H. Boehringer v. 1912 beschrieben.

Meptazinol (3-(3-ethyl hexa hydro-1-methyl-1H-azepin-3-yl)phenol), ein Nakotikum und Analgetikum, wird in der DE-OS 1 941 534 oder GB 1 285 025 beschrieben.

Tilidin (trans- 2- (di methyl amino)- 1- phenyl- 3- cyclo hexene- 1- carboxylic acid ethyl ester) wird in der DE 1 518 959 oder der US 3 557 126 beschrieben und ist ein bekanntes Analgetikum und Narkotikum.

In einer erfindungsgemäßen und bevorzugten Ausfürungsform wird Codein verwendet, vorzugsweise in Form der freien Base, des HBr- oder HI-Salzes oder als Codein-Phosphat.

In einer erfindungsgemäßen und bevorzugten Ausfürungsform wird Tilidin verwendet, vorzugsweise in Form der freien Base oder des HCl-Salzes.

In einer erfindungsgemäßen und bevorzugten Ausfürungsform wird Meptazinol verwendet, vorzugsweise in Form der freien Base oder des HCl-Salzes.

Auch wenn die erfindungsgemässen Verwendungen lediglich geringe Nebenwirkungen zeigen, kann es beispielsweise zur Vermeidung von bestimmten Formen der Abhängigkeit auch von Vorteil sein, neben diesen Verbindungen auch Morphinantagonisten, insbesondere Naloxon, Naltrexon und/oder Levallorphan, zu verwenden. Ein bevorzugtes Beispiel wäre Tilidin und Naloxon.

Die Erfindung umfaßt ebenfalls Arzneimittel zur Behandlung von vermehrtem Harndrang bzw. Harninkontinenz, die als Wirkstoff wenigstens eine der Verbindungen ausgewählt aus
■ Codein,
■ Meptazinol oder
■ Tilidin
als freie Base und/oder in Form physiologisch verträglicher Salze sowie gegebenenfalls Zusatz- und/oder Hilfsstoffe enthalten. Geeignete Salze im Sinne dieser Erfindung und in jeder der beanspruchten Verwendungen sind Salze des jeweiligen Wirkstoffes mit anorganischen bzw. organischen Säuren und/oder einem Zuckeraustauschstoff wie Saccharin, Cyclamat oder Acesulfam. Besonders bevorzugt ist jedoch das Hydrochlorid.

Geeignete Zusatz- und/oder Hilfsstoffe im Sinne dieser Erfindung sind alle dem Fachmann aus dem Stand der Technik bekannten Stoffe zur Erreichung galenischer Formulierungen. Die Auswahl dieser Hilfsstoffe sowie die einzusetzenden Mengen derselben hängen davon ab, ob das Arzneimittel oral, intravenös, intraperitoneal, intradermal, intramuskulär, intranasal, buccal oder lokal appliziert werden soll. Für die orale Applikation eignen sich Zubereitungen in Form von Tabletten, Kautabletten, Dragees, Kapseln, Granulaten, Tropfen, Säften oder Sirupen, für die parenterale, topische und inhalative Applikation Lösungen, Suspensionen, leicht rekonstituierbare Trockenzubereitungen sowie Sprays. Eine weitere Möglichkeit sind Suppositorien für die Anwendung im Rektum. Die Anwendung in einem Depot in gelöster Form, einer Trägerfolie oder einem Pflaster, gegebenenfalls unter Zusatz von die Hautpenetration fördernden Mitteln, sind Beispiele für geeignete perkutane Applikationsformen. Beispiele für Hilfs- und Zusatzmitteln für die oralen Applikationsformen sind Sprengmittel, Gleitmittel, Binder, Füllmittel, Formtrennmittel, gegebenenfalls Lösungsmittel, Geschmacksstoffe, Zucker, insbesondere Trägermittel, Verdünnungsmittel, Farbstoffe, Antioxidantien etc. Für Suppositorien können u.a. Wachse bzw. Fettsäureester und für parenterale Applikationsmittel Trägerstoffe, Konservierungsmittel, Suspensionshilfsmittel etc. verwendet werden. Die an Patienten zu verabreichenden Wirkstoffmengen variieren in Abhängigkeit vom Gewicht des Patienten, von der Applikationsart und dem Schweregrad der Erkrankung. Aus oral, rektal oder perkutan anwendbaren Zubereitungsformen können die erfindungsgemäßen Verbindungen verzögert freigesetzt werden. Bei der erfindungsgemäßen Indikation sind entsprechende Retard-Formulierungen, insbesondere in Form eines "Once-daily"-Präparats, das nur einmal am Tag eingenommen werden muß, besonders bevorzugt.

Weiter bevorzugt sind Arzneimittel, die wenigstens 0,05 bis 90,0 % des Wirkstoffes enthalten, insbesondere niedrige wirksame Dosierungen, um Neben- oder analgetische Wirkungen zu vermeiden.

Bevorzugtermaßen werden bei der erfindungsgemäßen Verwendung der Stoffe für ein Arzneimittel für die Harninkontinenz bzw. für ein diese Wirkstoffe enthaltendes Arzneimittel im Arzneimittel eine Dosis eingesetzt, die niedriger ist als die für eine analgetische Wirkung notwendige, also Dosierungen unterhalb der analgetischen Wirkung verwendet. Üblich sind Dosierungen zwischen der Untergrenze der bei Schmerztherapie verwendeten Dosis und 10% dieser Dosis, vorzugsweise zwischen 80% und 20% dieser Dosis, insbesondere zwischen 50 und 30 %.

Für die Opioide, deren Verwendung in der Harninkontinenz erfindungsgemäß beansprucht wird, bedeutet das, das üblicherweise folgende konkreten Dosen pro Gabe eingesetzt werden:

| **Substanz** | **Humandosis [mg]** | | | **Dosis [µg/kg]** | | |
|---|---|---|---|---|---|---|
| | **üblich** | **bevorzugt** | **insbes.** | **üblich** | **bevorzugt** | **insbes.** |
| Codein | 4-40 | 8-32 | 12-20 | 62-616 | 123-493 | 185-308 |
| Meptazinol | 5-50 | 10-40 | 15-25 | 77-770 | 154-616 | 231-385 |
| Tilidin | 5-50 | 10-40 | 15-25 | 77-770 | 154-616 | 231-385 |

Hilfsstoffe können beispielsweise sein: Wasser, Ethanol, 2-Propanol, Glycerin, Ethylenglycol, Propylenglycol, Polyethylenglycol, Polypropylenglycol, Glucose, Fructose, Lactose, Saccharose, Dextrose, Melasse, Stärke, modifizierte Stärke, Gelatine, Sorbitol, Inositol, Mannitol, mikrokristalline Cellulose, Methylcellulose, Carboxymethylcellulose, Celluloseacetat, Schellack, Cetylalkohol, Polyvinylpyrrolidon, Paraffine, Wachse, natürliche und synthetische Gummis, Akaziengummi, Alginate, Dextran, gesättigte und ungesättigte Fettsäuren, Stearinsäure, Magnesiumstearat, Zinkstearat, Glycerylstearat, Natriumlaurylsulfat, genießbare Öle, Sesamöl, Kokusnußöl, Erdnußöl, Sojabohnenöl, Lecithin, Natriumlactat, Polyoxyethylen- und -propylen-fettsäureester, Sorbitanfettsäureester, Sorbinsäure, Benzoesäure, Citronensäure, Ascorbinsäure, Tanninsäure, Natriumchlorid, Kaliumchlorid, Magnesiumchlorid, Calciumchlorid, Magnesiumoxid, Zinkoxid, Siliciumdioxid, Titanoxid, Titandioxid, Magnesiumsulfat, Zinksulfat, Calciumsulfat, Pottasche, Calciumphosphat, Dicalciumphosphat, Kaliumbromid, Kaliumiodid, Talkum, Kaolin, Pectin, Crospovidon, Agar und Bentonit.

Die Herstellung der erfindungsgemäßen Arzneimittel und pharmazeutischen Zusammensetzungen erfolgt mit Hilfe von im Stand der Technik der pharmazeutischen Formulierung wohlbekannten Mitteln, Vorrichtungen, Methoden und Verfahren, wie sie beispielsweise in "Remington's Pharmaceutical Sciences", Hrsg. A.R. Gennaro, 17. Ed., Mack Publishing Company, Easton, Pa. (1985), insbesondere in Teil 8, Kapitel 76 bis 93, beschrieben sind.

So kann z.B. für eine feste Formulierung, wie eine Tablette, der Wirkstoff des Arzneimittels, d.h. eine Verbindung der gegenwärtigen Anmeldung oder eines ihrer pharmazeutisch annehmbaren Salze, mit einem pharmazeutischen Träger, z.B. herkömmlichen Tabletteninhaltsstoffen, wie Maisstärke, Lactose, Saccharose, Sorbitol, Talkum, Magnesiumstearat, Dicalciumphosphat oder pharmazeutisch akzeptable Gummis, und pharmazeutischen Verdünnungsmitteln, wie z.B. Wasser, granuliert werden, um eine feste Zusammensetzungzu bilden, die eine erfindungsgemäße Verbindung oder ein pharmazeutisch annehmbares Salz davon in homogener Verteilung enthält. Unter einer homogenen Verteilung wird hier verstanden, daß der Wirkstoff gleichmäßig über die gesamte Zusammensetzung verteilt ist, so daß diese ohne weiteres in gleich wirksame Einheitsdosis-Formen, wie Tabletten, Pillen oder Kapseln, unterteilt werden kann. Die feste Zusammensetzungwird anschließend in Einheitsdosis-Formen unterteilt. Die Tabletten oder Pillen des erfindungsgemäßen Arzneimittels bzw. der erfindungsgemäßen Zusammensetzungen können auch überzogen oder auf andere Weise kompoundiert werden, um eine Dosisform mit verzögerter Freisetzung bereitzustellen. Geeignete Beschichtungsmittel sind u.a. polymere Säuren und Mischungen von polymeren Säuren mit Materialien wie z.B. Schellack, Cetylalkohol und/oder Celluloseacetat.

Die folgenden Beispiele sollen die Erfindung erläutern.

### Beispiele

### Beispiel 1: Testsystem Cystometrie an der narkotisierten naiven Ratte

Die cystometrische Untersuchung an naiven weiblichen Ratten wurde nach der Methode von Kimura et al. (Kimura et al., 1996, Int. J. Urol. 3:218-227) durchgeführt. An narkotisierten, ventilierten Ratten wird das Abdomen eröffnet und die Harnleiter abgebunden. Der Harn wird von den Nieren abgeleitet. Ein Katheter wird in die Blase eingeführt und fixiert. Über diesen wird Saline mittels Infusionspumpe in die Blase infundiert, bis diese rhythmische Spontanaktivität in Form von Kontraktionen zeigt, welche über einen angeschlossenen Druckaufnehmer aufgenommen werden können. Die Testsubstanz wird nach Erreichen stabiler Ausgangswerte in kumulativer Weise i.v. appliziert. Eine Beeinflussung der Blasenfunktion äußert sich über die Unterdrückung der Spontankontraktionen. Dabei gilt als Parameter für die Unterdrückung das Ausbleiben der Kontraktionen über einen Zeitraum von 10 min.

Bei allen hier aufgelisteten Substanzen war eine Unterdrückung der Spontankontraktionen in den Ratten meßbar, wobei die Tabelle 1 den Mittelwert der niedrigsten Dosis aus 3 unabhängigen Experimenten angibt, bei der erstmals Kontraktionen über einen Zeitraum von 10 min ausbleiben.

**Tabelle 1:**

| (n entspricht der Anzahl der in den Wert eingegangenen Versuche) | |
|---|---|
| **Verbindung** | **Niedrigste Dosis** **(mg/kg)** |
| **Tilidin** | 0,5 (n=3) |
| **Meptazinol** | 1,0 (n=3) |
| **Codein**(Phosphat) | 4,7 (n=3) |

Die untersuchten Substanzen zeigen eine positive Wirkung auf die Blasenregulation und sind somit geeignet zur Behandlung der Harninkontinenz.

### Beispiel 2: Parenterale Applikationsform

38,5 g Meptazinol-HCl wird in 1 l Wasser für Injektionszwecke bei Raumtemperatur gelöst und anschließend durch Zugabe von wasserfreier Glukose für Injektionszwecke auf isotone Bedingungen eingestellt. Appliziert werden davon bei einem Durchschnittspatienten von ca. 65 kg Körpergewicht beispielsweise 0,5 ml also 19,25 mg bzw. ≈ 300 µg/kg. Die Gabe kann etwa 3 mal täglich erfolgen.

### Beispiel 3: Flüssige orale Applikationsform als Kombination mit Naloxon

23,82 g Tilidin-HCl ½ H₂O und 2,04 g Naloxon-HCl 2H₂O werden in 874 ml gereigtem Wasser, 124 ml Ethanol (96%) und 2 ml HCl bei Raumtemperatur gelöst.

Davon werden bei einem Durchschnittspatienten von ca. 65 kg Körpergewicht beispielsweise 20 Tropfen (≈ 0,72 ml) eingenommen also 17,15 mg Wirkstoff (entspricht ca. 16,7 mg Tilidin-HCl) bzw. 264 µg/kg. Das kann insgesamt bis zu 4 mal täglich erfolgen.

### Beispiel 4: Feste orale retardierte Applikationsform (Retardtabletten)

| Pro Retardtablette | | |
|---|---|---|
| | 17 mg | Codeinphospat ½ H₂O |
| | 143 mg | Mikrokristalline Cellulose |
| | 94 mg | Methylhydroxypropylcellulose 100 000 mPa·s |
| | 3 mg | Hochdisperses Siliziumdioxid |
| | 3 mg | Magnesiumstearat |
| | 260 mg | Gesamt |

Die Hilfsstoffe und der Wirkstoff werden in einem Mischer homogen gemischt und anschließend auf einer Tablettenpresse zu Tabletten mit einem ⌀ von 9 mm verpreßt.

Beispiel für eine Dosis: 1 Tablette, also beim Körpergewicht von 65kg ≈ 262 µg/kg. Üblicherweise werden 2 Tabletten am Tag eingenommen.

## Patentansprüche

1. Verwendung einer der folgenden Verbindungen
■ Codein,
■ Meptazinol oder
■ Tilidin
zur Herstellung eines Arzneimittels zur Behandlung von vermehrtem Harndrang bzw. Harninkontinenz.

2. Verwendung gemäß Anspruch 1, **dadurch gekennzeichnet, daß** Codein, vorzugsweise in Form der freien Base, des HBr- oder HI-Salzes oder als Codein-Phosphat, verwendet wird.

3. Verwendung gemäß Anspruch 1, **dadurch gekennzeichnet, daß** Tilidin, vorzugsweise in Form der freien Base oder des HCl-Salzes, verwendet wird.

4. Verwendung gemäß Anspruch 1, **dadurch gekennzeichnet, daß** Meptazinol, vorzugsweise in Form der freien Base oder des HCl-Salzes, verwendet wird.

5. Verwendung gemäß einem der Ansprüche 1 bis 4, **dadurch gekennzeichnet, daß** im Arzneimittel folgende Dosen der Wirkstoffe verwendet werden:
a) 4-40 mg, vorzugsweise 8-32 mg, insbesondere 12-20 mg Codein,
b) 5-50 mg, vorzugsweise 10-40 mg, insbesondere 15-25 mg Meptazinol oder
c) 5-50 mg, vorzugsweise 10-40 mg, insbesondere 15-25 mg Tilidin.

## Claims

1. Use of one of the following compounds
• codeine,
• meptazinol or
• tilidine
for the preparation of a medicament for treatment of an increased urge to urinate or urinary incontinence.

2. Use according to claim 1, **characterized in that** codeine, preferably in the form of the free base, the HBr or HI salt or as codeine phosphate, is used.

3. Use according to claim 1, **characterized in that** tilidine, preferably in the form of the free base or the HCl salt, is used.

4. Use according to claim 1, **characterized in that** meptazinol, preferably in the form of the free base or the HCl salt, is used.

5. Use according to one of claims 1 to 4, **characterized in that** the following doses of the active compounds are used in the medicament:
a) 4-40 mg, preferably 8-32 mg, in particular 12-20 mg of codeine,
b) 5-50 mg, preferably 10-40 mg, in particular 15-25 mg of meptazinol or
c) 5-50 mg, preferably 10-40 mg, in particular 15-25 mg of tilidine.

## Revendications

1. Utilisation de l'un des composés suivants
• codéine
• meptazinol ou
• tilidine
pour la préparation d'un médicament destiné au traitement du besoin accru d'uriner ou de l'incontinence urinaire.

2. Utilisation suivant la revendication 1, **caractérisée en ce que** la codéine est utilisée avantageusement sous forme de la base libre, du sel de HBr ou de HI ou sous forme de phosphate de codéine.

3. Utilisation suivant la revendication 1, **caractérisée en ce que** la tilidine est utilisée avantageusement sous forme de la base libre ou du sel de HCl.

4. Utilisation suivant la revendication 1, **caractérisée en ce que** le meptazinol est utilisé avantageusement sous forme de la base libre ou du sel de HCl.

5. Utilisation suivant l'une des revendications 1 à 4, **caractérisée en ce qu'**on utilise dans le médicament les doses suivantes de substances actives :
a) 4 à 40 mg, avantageusement 8 à 32 mg, notamment 12 à 20 mg de codéine,
b) 5 à 50 mg, avantageusement 10 à 40 mg, notamment 15 à 25 mg de meptazinol, ou
c) 5 à 50 mg, avantageusement 10 à 40 mg, notamment 15 à 25 mg de tilidine.
